(19) Europäisches Patentamt
European Patent Office
Office européen des brevets

(11) EP 3 075 851 B1

(12) **EUROPEAN PATENT SPECIFICATION**

(45) Date of publication and mention of the grant of the patent:
**06.12.2017 Bulletin 2017/49**

(51) Int Cl.:
***C12N 15/09*** *(2006.01)* ***C12Q 1/04*** *(2006.01)*
***C12Q 1/68*** *(2006.01)*

(21) Application number: **16163065.2**

(22) Date of filing: **31.03.2016**

(54) **METHOD FOR ACQUIRING INFORMATION ON GASTRIC CANCER AND KIT FOR DETECTION OF GASTRIC CANCER**

VERFAHREN ZUR INFORMATIONSERFASSUNG ÜBER MAGENSAFT UND KIT ZUM NACHWEIS VON MAGENKREBS

PROCÉDÉ POUR ACQUÉRIR DES INFORMATIONS SUR LE CANCER GASTRIQUE ET KIT POUR LA DÉTECTION DU CANCER GASTRIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.03.2015 JP 2015073624**

(43) Date of publication of application:
**05.10.2016 Bulletin 2016/40**

(73) Proprietors:
- **SYSMEX CORPORATION**
  **Kobe-shi,**
  **Hyogo 651-0073 (JP)**
- **The University of Tokyo**
  **Bunkyo-ku,**
  **Tokyo 113-8654 (JP)**

(72) Inventors:
- **Tai, Kaya**
  **Kobe-shi**
  **Hyogo 651-0073 (JP)**
- **Nagae, Genta**
  **Tokyo 113-8654 (JP)**
- **Aburatani, Hiroyuki**
  **Tokyo 113-8654 (JP)**

(74) Representative: **Paemen, Liesbet R.J. et al**
**De Clercq & Partners**
**Edgard Gevaertdreef 10 a**
**9830 Sint-Martens-Latem (BE)**

(56) References cited:
**WO-A1-2013/097868** **WO-A1-2014/062218**
**WO-A2-2012/070861**

- **JAN K M ET AL: "Abnormal DNA methylation according to the histologic types of early gastric adenocarcinoma", HISTOPATHOLOGY, WILEY-BLACKWELL PUBLISHING LTD, GB , vol. 61, no. Supplement 1 5 September 2012 (2012-09-05), pages 76-77, XP008180352, ISSN: 0309-0167, DOI: 10.1111/J.1365-2559.2012.04359_10.X Retrieved from the Internet: URL:http://onlinelibrary.wiley.com/doi/10. 1111/j.1365-2559.2012.04359_10.x/pdf [retrieved on 2012-08-30]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 075 851 B1

SEQUENCE LISTING

<110> SYSMEX CORPORATION
THE UNIVERSITY OF TOKYO

<120> Method for acquiring information on gastric cancer and kit for detection of gastric cancer

<130> SYSM-103-EP

<150> JP 2015-073624
<151> 2015-03-31

<160> 7

<170> PatentIn version 3.5

<210> 1
<211> 4120
<212> DNA
<213> Homo sapiens

<400> 1

```
aaaaatgagt aaagatttga actgacatgt cacaaaagat gactaaaaac caataaacat      60
atggaatatg ttcaatttta ttattaatca cggaaaccca aattcaaacc tgtgggggtg     120
ggcgggtata agtacagata tatattcaaa tgaggattta aaagtctggc atgtctgatt     180
tgtatatcat agtttttgca aacatcaaaa atgtatttcc caagaccatt tgatgacatg     240
ggagaatatt tctgacactt ctttttgcaa aaatgtagaa catcaaatgg taaatgaaat     300
aaaatttggt ttttgacaaa taatgaaaat acattaaaat attaacccta gttatctgag     360
tggctaaatt ttattttttc ttggtaatgc tgtgtttttg agatctctaa attaatcatc     420
cttttatttt taaatcagaa taaatatttg agaagaaaat tcagcaagca taagtggagc     480
aaccatcctt ttctgtgatg ggaaaattat agggacctat aattaaatga cattccttta     540
agcaagaatt ttcagaagaa aaagaggaaa ccccaacaca ccttggatac tgcttgtaga     600
agttcatcat gcattctttc ctcctcttct gggtgacacc tggagaaagg taaaattggg     660
agagggaaga gtaactgtga gacaccaggt ttttcttggt acccagatta gtcaagtaag     720
agttcttccc cagccccatt acccaccaca catgaggaat ggagatcaga ggagaatggg     780
gcattctgtc caattccagt ggctcagggg aaaggttgtg ttcactgctg tccagggttg     840
gaatctgacc agacctcaga gaggtttcct agagacagag ttctggagtc acaatcgcat     900
agttgacagg accccatact catacacaga atgccacata catttaggga cagtcacact     960
taaggcgaca tgcacgtctc acctgcccac aaggagccac acacggcaac acagttacaa    1020
tcacatacac accataggaa acacacaatt acagctgcac aatcgtaggc accccacact    1080
gtcgcagagt cacaatcaca catcaccacg ggaacacaca gccacaaccc ccacagtgac    1140
aggtatccca tatccgcaca taagccgcag tgttattatc tcccagacaa tttcacaacc    1200
```

tcccctcttt ttttgggggg gtggggtggg gtggggacag agtcttgccc tgtcgccagg 1260

ctggagtgca gtggtgcgat ctcggctcac tgcaacctcc gcctcccggg ttcaagtgat 1320

tctcctgcct cagcctgcga gtagctggga ttacaggcgc gcgccaccat gcctagctaa 1380

tttttgtatt tttagtagag atggggtttc accatgttgg ccaggatggt ctcgatctct 1440

tgacctcgtg atcctcctgc ctcggcctcc caaacacaac ctccaccttt caggccgctc 1500

cgcccgcagg ggcccaaggc gccagctccc catcggtctt cggtatcctc acctaagccc 1560

tttccagtca tcccggatgg gaacttgcag acctgagcca gttctcctgg ggaccaaaat 1620

atctcacctc ccagatctaa gggtcccgcc aggagtgacg aaacgttcga attcctgcga 1680

gaaaagtggc aggccaccag gccctctggg aaatgtagtc cagagcggga cccacgccga 1740

ttcctgtcag ctcctcgcct gggcccaccc gaaacggctg ctccctcaac tctcaacatc 1800

cagccgagcc tcggagttgc gggtcgccgt agcgctgcgc aatggagatg agcctcccgg 1860

ggaacccggc ccaagcctca ccctcacaca ggaaagcaga tgtgttctgg ccggaagttg 1920

agtggggccg cggggcctgc tgggaggtgt tgtcctcgga aacgtcgctg gcgcggaggg 1980

atggttcggc gctttaggcg tctgtcacag acctatctgc gggtcgcctt cacccagcat 2040

ctcagaaact gcgcgcggga tgaacattcg ggtgtttccg gcaggtgacg ctgccgagtc 2100

cccgcagcag gggcgagca agggactcgc ggttgacggg acacggatcc tctaaggccc 2160

agagtgtccc gagtagcggc agtggggagt gctcagggta cgctaatggt gagtggttgc 2220

agttgatggg acaaaaaact gtgatgggag ttagtgtggg tgtgtggttg tgtgtgtgag 2280

tgtgacggca cgaataatct gatggggtga ttgtgatatt tggttgcgcg ggattatgat 2340

gaaatgtgtg atatgtgtgt gattatgatt aaggctgtag cgagtgtgaa cgtggcgaag 2400

tgtgcgtgat tgtaatatgt ctagtagttc ttgtcttgag ctggcttttc tgtagcattt 2460

ggcacagttg gtaacggctt gaaaaacact tatttgtttt ctactgatcc tccctccttg 2520

ttggccactc ctctgtagtc ttctttgctg tctttttac cctagtagcc cttggccctt 2580

tgtaattttc cactattcct tggcgaactt ttcaggttca ttattatctt atgacttgct 2640

gcttttatgt atgttagtta gctattgcca tgataataat tatgtctaaa aaccacccct 2700

gaactcaatg ccatacaaca gcaagtcctt actctcctgt tcacaggctt gcgcattgac 2760

tgtgttcagc tgatctaggt ggggcttggc tgagtggata ggctgcgtga cgagttaaga 2820

tctgcgccgc atgtgttctt tcagggtctc aggccaaaag gacatttgtt gccagggcat 2880

gctctgctcc tgatgggtca ccagagcgca agagagattg cctcaaccag agcaagtcac 2940

atggctaagc ctaaaatcaa ttatgcccac agttggagag caaagttacc tcgcaaagag 3000

tatgaataat aatctattag agggtgtgag gaattggact aaaattccag tctaccacct 3060

taagaaacat acctctttat cctggtgaat gccttttagt ccgggttctc ttttgtctga 3120

```
taatattgct tttcaagttt tattgtattt cttaagaatt tcattttcta accccccttgt     3180
gtctttatgt ttcagatctg tgtcttgtaa agagcatatt gctgggctta catccgttta     3240
tcttgtttga ctgttatttt aataggtgag tatagttcag tggcatttat catgattgct     3300
cataaaaaat gttttatttt ctattacgcc ttaaacattt ttgattgaca tataatagtt     3360
gcacatatat cttttttat tgtgcaaatt caccttatcc ctttttctcc cctaggttgg     3420
tttctgctat tatccttttt taatatgcat gcttaacaaa actcaacctt aagactttaa     3480
aatacttgaa ctccaaaata tccaccaatt tctgtgttat tcttgtctca tatattattt     3540
tcttctagtt tttgttgttg ttgttgtttt gttttgtttt tttgagacgg agtctcgctc     3600
tgtcacccag gctggagtgc agtggcacgg tctcggctca ctgcaaactc cgcctcccga     3660
gttcaagcga ttctcctgcc tcagcctccc gagtagctgg gactacagac gcgccaccac     3720
gcccggctga ttttttgtat ttttagcaga gacagggttt caccatgtta gccaccatgg     3780
tagatctcct gacctcgtga tttgccgacc tcagcctccc aaagtgctgg gattacaggc     3840
gtaagccacc atgcccagcc tattctcttc tagttttaaa tccccaaatt agtagtagta     3900
gctcatttat tctatataca gttcgtatcc aggaggggtt ggtttcaaga ccccccatgg     3960
ataccagaat ccacagatgc tcaagtccct acatccttct ttatacatta aatcatctct     4020
agattatttg taatacctaa tataatgtaa atgctgtgta aatcattgtt acacgttatt     4080
gtttagggaa taatgagaaa aaaatctgta ctgtacgtgt                           4120
```

<210> 2
<211> 120
<212> DNA
<213> Homo sapiens

<400> 2

```
tctgtcacag acctatctgc gggtcgcctt cacccagcat ctcagaaact gcgcgcggga       60
tgaacattcg ggtgtttccg gcaggtgacg ctgccgagtc cccgcagcag ggggcgagca      120
```

<210> 3
<211> 120
<212> DNA
<213> Artificial Sequence

<220>
<223> The sequence converted with bisulfites from the amplified region in ZNF promotor.

<400> 3

tutgtuauag auutatutgc gggtcguutt uauuuaguat utuagaaaut gcgcgcggga 60

tgaauattcg ggtgtttucg guaggtgacg utgucgagtu uucguaguag ggggcgagua 120

<210> 4
<211> 25
<212> DNA
<213> Artificial Sequence

<220>
<223> Forward M primer for amplification of the region in ZNF promotor.

<400> 4
tttgttatag atttatttgc gggtc 25

<210> 5
<211> 20
<212> DNA
<213> Artificial Sequence

<220>
<223> Reverse M primer for amplification of the region in ZNF568 promotor.

<400> 5
tactcgcccc ctactacgaa 20

<210> 6
<211> 33
<212> DNA
<213> Artificial Sequence

<220>
<223> Forward primer for control

<400> 6
gggatattaa gtggagttat tttggtttta gtt 33

<210> 7
<211> 21
<212> DNA
<213> Artificial Sequence

<220>
<223> Reverse primer for control

<400> 7
ccctcccaac atccttccta a 21

## Description

TECHNICAL FIELD

The present invention relates to a method for acquiring information on gastric cancer of a subject and a kit for detection of gastric cancer.

BACKGROUND

Gastric cancer is, next to lung cancer, known as causing the second largest number of deaths. Gastric cancer examination includes stomach endoscopy and stomach X-ray examination. However, the stomach endoscopy sometimes induces a vomiting reflex at the time of insertion of a stomach camera, and the stomach X-ray examination requires drinking of barium sulfate. Therefore, these examinations impose a big burden on subjects.

In recent years, methods based on genetic information have been studied as new methods for diagnosing cancer. Examples of such methods include a method based on information on the methylation of DNA. This method employs a CpG site (5'-(CG)-3') in the base sequence of a predetermined gene as a marker. Information, for example, on the presence or absence of cancer cells is acquired based on the analysis results of the methylation state of the marker, and this information is employed as an index for diagnosis of cancer.

For example, WO 2013/097868 and WO 2014/062218 describe ZNF568 genes as one of methylation markers for colorectal cancer. Also, Hrasovec S. et al., Disease Markers 34 (2013) p. 93-104 describes ZNF568 as a highly-methylated gene in colorectal cancer. For example WO 2012/070861 describes methylation markers for gastric cancer.

SUMMARY OF THE INVENTION

While it has been reported that ZNF568 genes exhibit abnormal methylation in colorectal cancer as described above, neither of these documents refer to correlation between ZNF568 genes and gastric cancer. On the other hand, there are a small number of genes that can be used as markers for detection of gastric cancer. Therefore, there is a demand for further development of a novel marker for detection of gastric cancer utilizing methylation analysis of genes.

The present inventors have identified, as novel markers, gene regions where methylation is observed specifically for DNA obtained from gastric cancer part tissues. Then, the present inventors have found that cancer cells derived from gastric cancer and other cells (normal tissue cells, non-cancer part tissue cells, and cancer cells derived from different types of cancer from gastric cancer, except colorectal cancer) can be clearly discriminated based on the results obtained by analysis of the methylation state of these markers. Then, the present inventors completed the present invention.

Specifically, the present invention provides an in vitro method for acquiring information on gastric cancer in a subject. The method comprises the steps of: analyzing in a DNA specimen of a biological sample of said subject, the methylation status of a CpG site in a promoter region of ZNF568 gene; and acquiring, based thereon, information that the biological specimen comprises a gastric cancer cell. More particularly, the method comprises acquiring information that the biological sample comprises a gastric cancer cell when it is determined that the methylation is present.

In particular embodiments, the method comprises the steps of preparing a DNA specimen from a biological sample collected from a subject; analyzing methylation status of a CpG site in a promoter region of ZNF568 gene contained in the DNA specimen; and acquiring information that the biological sample comprises a gastric cancer cell when it is determined that the methylation is present. In particular embodiments, the methylation status of SEQ ID NO: 2 is analyzed in the analyzing step. In particular embodiments, the analyzing step is conducted by at least one method selected from the group consisting of mass spectrometry and methylation-specific PCR. In particular embodiments, the biological sample is a tissue, blood or serum. In particular embodiments, the analyzing step comprises: calculating methylation frequency of the promoter region, and the acquiring step comprises: comparing the frequency with a predetermined threshold; and acquiring information that the biological specimen comprises the gastric cancer cell when the frequency is equal to or higher than the threshold. In particular embodiments, In particular embodiments, the method comprises the step of preparing the DNA specimen from a biological sample of said subject, and said preparing step comprises treating DNA in the biological specimen with bisulfite.

The invention further relates to the use of a reagent kit for the detection of gastric cancer, said kit comprising a primer capable of hybridizing to a promoter region of ZNF568 gene, wherein said promoter region comprises: a uracil base converted from unmethylated cytosine base by a bisulfite treatment and methylated cytosine base, wherein the primer is used for analyzing methylation status of a CpG site in said promoter region of ZNF568 gene contained in a DNA specimen. In particular embodiments, the primer is a primer consisting of the base sequence of SEQ ID NO: 4 or 5.

The present invention can provide a novel marker for detection of gastric cancer.

BRIEF DESCRIPTION OF THE DRAWINGS

Fig. 1 is a graph showing the methylation positive rate of the promoter region of ZNF568 gene calculated from methylation data for cancer part tissues and non-cancer part tissues of gastric cancer and normal tissues.
Fig. 2 is a graph showing the methylation positive rate of the promoter region of ZNF568 gene calculated from methylation data for various clinical samples.
Fig. 3 is a photograph showing results of amplification of DNA specimens extracted from gastric cancer and normal stomach tissues by methylation-specific PCR (MSP method).
Fig. 4 is a schematic view showing one example of a determination device for providing information on gastric cancer of a subject.
Fig. 5 is a block diagram showing the function configuration of the determination device shown in Fig. 4.
Fig. 6 is a block diagram showing the hardware configuration of the determination device shown in Fig. 4.
Fig. 7 is a flow chart of determination for providing the information on gastric cancer of the subject using the determination device shown in Fig. 4.

DETAILED DESCRIPTION OF THE PREFERRED EMBODIMENTS

The method for acquiring information on gastric cancer as envisaged herein (hereinafter also referred to merely as the "method") is carried out on a DNA specimen prepared from a biological sample of the subject. In particular embodiments, the method also comprises the step of the preparation of a DNA specimen from a biological sample collected from a subject.

In the methods envisaged herein, the biological sample is not particularly limited so long as the sample is an organism-derived sample including DNA of the subject. Preferably, the biological sample is a sample including genome DNA, for example, a clinical sample. Examples of the clinical sample include body fluid, urine, and tissues collected through surgery or biopsy. Examples of the body fluid include blood, serum, plasma, lymph, ascites, bone marrow fluid, and nipple secretion. Cultures obtained by culturing cells or tissues collected from the subject can also be used as biological samples or specimens. Formalin-fixed paraffin embedded (FFPE) specimens of tissues collected from the subject may be used as biological samples.

The DNA specimen can be prepared by extracting DNA from biological samples. Methods for extracting DNA from biological samples are known in the art. For example, DNA can be extracted by mixing biological specimens and a treatment solution containing a surfactant (for example, sodium cholate or sodium dodecyl sulfate) which solubilizes cells or tissues, and subjecting the resultant solution mixture to physical treatment (for example, stirring, homogenization or ultrasonic crushing) to liberate the DNA contained in the biological samples in the solution mixture. In this case, it is preferred to centrifuge the solution mixture to precipitate cell fragments and to use the supernatant containing the liberated DNA for an analysis step as will be described below. The resultant supernatant may be purified by a known method in the art. The DNA from the biological sample can also be extracted and purified using a commercial kit.

The above-described preparation step preferably further includes the step of fragmenting the extracted DNA. Fragmentation of the DNA into an appropriate length ensures effective methylated DNA immunoprecipitation (MeDIP) method and non-methylated cytosine conversion treatment which will be described below.

DNA can be fragmented, for example, by ultrasonication, alkali treatment or restriction enzyme treatment. For example, in the fragmentation of DNA by alkali treatment, DNA is fragmented by adding a sodium hydroxide solution to a DNA solution so as to attain a final concentration of 0.1N to 1.0N and incubating it at 10°C to 40°C for 5 minutes to 15 minutes. The restriction enzyme used in the fragmentation of DNA by restriction enzyme treatment is appropriately selected based on the base sequence of the DNA. For example, MseI or BamHI is used as the restriction enzyme.

The method as envisaged herein involves determining the presence or absence of the methylation of a CpG site that is present in the promoter region of ZNF568 (zinc finger protein 568) gene contained in the DNA specimen of the subject . The term "CpG site" as used herein means a site of a base sequence wherein cytosine (C) and guanine (G) are adjacent in this order in the 5' $\rightarrow$ 3' direction. The letter "p" of CpG represents a phosphodiester bond between cytosine and guanine.

The base sequence of the promoter region of ZNF568 gene is *per se* known in the art. The base sequence can be known from known database such as the database provided by the National Center for Biotechnology Information (NCBI) in the United States National Library of Medicine (http://www.ncbi.nlm.nih.gov/). Various ID numbers of ZNF568 gene are indicated in Table 1. The base sequence of the promoter region of ZNF568 gene is indicated by SEQ ID NO: 1.

[Table 1]

| Gene name | Unigene ID | Entrez Gene ID | SEQ ID NO: |
|---|---|---|---|
| ZNF568 | 218494 | 374900 | 1 |

In particular embodiments, the presence or absence of the methylation of a CpG site can be determined based on the results of methylation analysis of the above-described promoter region. The methylation analysis can be performed, for example, by investigating whether or not cytosine at at least one CpG site, among CpG sites that are present in the promoter region of ZNF568 gene, has been methylated. In this case, one CpG site may be analyzed. However, the presence or absence of the methylation of a plurality of CpG sites is preferably analyzed.

The plurality of CpG sites may be either selected from the promoter region of one gene or selected from the respective promoter regions of a plurality of genes.

In particular embodiments, methylation analysis may be performed by investigating the frequency of methylation in the promoter region of ZNF568 gene. The "frequency of methylation" can be, for example, a proportion of the number of methylated CpG sites to the number of CpG sites that are present in the above-described promoter region. In these embodiments, the target for analysis may be either the entire promoter region described above or a portion thereof including at least one CpG site. The target for analysis may include only one CpG site. However, the target for analysis preferably includes a plurality of CpG sites. The target for analysis may be either determined from the promoter regions of any one of the genes described above or determined from the promoter region of the plurality of genes. Since the positions and number of the CpG sites that are present in the promoter region of ZNF568 gene are known, the number of the methylated CpG sites in the promoter region, itself, can be utilized as the frequency of methylation.

The frequency of methylation described above may be "methylation score" obtained by analysis of the methylation state of a CpG site in the DNA by mass spectrometry such as MassARRAY (registered trademark) as will be described below. MassARRAY (registered trademark) ensures measurement of DNA fragments to calculate the methylation score from an area ratio between the peak derived from methylated DNA fragments and the peak derived from non-methylated DNA fragments.

In the above described embodiments, the frequency of methylation in the promoter region of ZNF568 gene may be calculated either by a hand method or by means of a computer or other machines.

In the methods provided herein, it is not particularly limited which CpG sites in the promoter regions of ZNF568 genes (or predetermined ranges including the CpG sites) are to be analyzed, and those skilled in the art can appropriately determine the target CpG sites (or ranges). The position and number of the CpG sites that are present in the promoter regions of these genes are known. Thus, the CpG sites or ranges to be analyzed can be determined through routine experiments using a known analysis method as will be described below.

Various analysis methods are known in the art as suitable means for methylation analysis. In the method provided herein, the analysis method to be used is not particularly limited. Preferably, a method is used which includes the steps of discriminating between methylated DNA and non-methylated DNA, amplifying the DNAs and detecting the methylated DNA and/or the non-methylated DNA.

Examples of the step of discriminating between methylated DNA and non-methylated DNA include steps of carrying out methylation-sensitive restriction enzyme treatment, MeDIP method, and non-methylated cytosine conversion treatment.

Examples of the step of amplifying the DNA include steps of carrying out PCR, quantitative PCR, IVT (in vitro transcription) amplification method, and SPIA™ amplification method.

Examples of the step of detecting the methylated DNA and/or the non-methylated DNA include steps of carrying out electrophoresis method, sequence analysis method, microarray analysis method, mass spectrometry, and Southern hybridization method.

The MeDIP method is a method involving concentrating methylated DNA contained in biological specimens by immunoprecipitation using an anti-methylated cytosine antibody or an anti-methylated cytidine antibody, or an antibody which specifically recognizes methylated DNA binding protein. In particular embodiments, the methylated DNA contained in the DNA obtained in the extraction step may be concentrated by the MeDIP method for methylation analysis of the methylated DNA obtained. Also, the methylated DNA concentrated by the MeDIP method can be amplified, for example, by the IVT amplification method for methylation analysis of the resultant amplification product using microarrays. Such an analysis method is referred to as MeDIP-on-chip method.

The non-methylated cytosine conversion treatment is treatment involving reacting DNA extracted from biological specimens and a non-methylated cytosine conversion agent to convert the non-methylated cytosine in the DNA into another base (uracil, thymine, adenine or guanine). The non-methylated cytosine conversion agent is a substance that can be reacted with DNA to convert non-methylated cytosine in the DNA into another base (uracil, thymine, adenine or guanine). For example, bisulfites including sodium, potassium, calcium and magnesium salts of hydrogen sulfite are suitably used as such a non-methylated cytosine conversion agent.

In the treatment of DNA with a bisulfite, the non-methylated cytosine in the DNA is converted into uracil through deamination reactions, but no such base conversion takes place in methylated cytosine. Thus, the difference in methylation state among CpG sites in DNA is converted into the difference in base sequence (whether C or U) by non-methylated cytosine conversion treatment with a bisulfite. Such non-methylated cytosine conversion treatment with a bisulfite is referred to as bisulfite treatment.

In the case of bisulfite treatment, the amount (concentration) of a bisulfite to be added is not particularly limited so long as it is enough to convert non-methylated cytosine in DNA. For example, the final concentration of the bisulfite in a solution containing DNA is 1M or more, preferably 1M to 15M, more preferably 3M to 10M. The conditions (temperature and time) for incubation after addition of the bisulfite can appropriately be set according to the amount of the bisulfite to be added. For example, when the bisulfite is added in a final concentration of 6M, incubation is conducted at 50°C to 80°C for 10 minutes to 90 minutes.

The methylation of CpG sites contained in DNA can be analyzed by sequence-analyzing the bisulfite-treated DNA to detect a difference from the original base sequence. This method is referred to as a bisulfite sequence method.

The methylation of CpG sites can be analyzed by mass spectrometry. Specifically, the PCR amplification using bisulfite-treated DNA as a template and a primer set specific for the base sequence to be analyzed is followed by further IVT amplification of the resultant PCR product so that methylated cytosine and uracil turn into guanine (G) and adenine (A), respectively. The resultant IVT amplification product is cleaved by RNase A, and the mass difference (16 Da) between G and A among the resultant nucleic acid fragments can be detected using a MALDI-TOF (Matrix Assisted Laser Desorption Ionization - Time of Flight) type mass spectrometer, thereby analyzing the methylation of DNA. This method is referred to as MassARRAY (registered trademark) analysis.

The site to be cleaved by RNase A in the IVT product is known to be located between an arbitrary base and uracil (U) or thymine (T) adjacent to the base. Thus, the base sequence and mass of the IVT product cleaved by RNase A can be predicted from the base sequence of the DNA used as a template. Therefore, the portion of the base sequence of the template DNA, from which the respective peaks obtained by MassARRAY (registered trademark) are derived, can be identified. For example, in the case of one methylated CpG site in a DNA fragment, the peak obtained by MassARRAY (registered trademark) is shifted, by 16 Da, to the high mass side. In the analysis of a DNA fragment having a plurality of CpG sites, for example, the peak is 32 Da shifted in the case of two methylated CpG sites in the DNA fragment, and 48 Da shifted in the case of three methylated CpG sites therein.

In the mass spectrometry such as MassARRAY (registered trademark), the methylation score of the DNA fragments which have been measured can be calculated. For example, when a DNA fragment having a predetermined sequence has an area ratio between the peak derived from non-methylated DNA fragments and the peak derived from methylated DNA fragments, in the chart obtained by analysis, of 1:3, the methylation score of this DNA fragment is 3/(1 + 3) = 0.75. Theoretically, the methylation score is 1 when all the CpG sites possessed by the DNA fragment have been methylated, and is 0 when none of the CpG sites have been methylated.

The methylation of CpG sites can be analyzed by a methylation-specific PCR (MSP) method. The MSP method is a method involving PCR-amplifying bisulfite-treated DNA using a primer set which will be described below to confirm the presence or absence of a PCR product, thereby analyzing the presence or absence of the methylation of CpG sites. The MSP method employs a primer set which allows for amplification of base sequences wherein the CpG sites to be analyzed have been methylated (namely, cytosine has not been converted into uracil), but which does not allow for amplification of base sequences wherein the CpG sites have not been methylated (namely, cytosine has been converted into uracil). It can be understood that the CpG sites to be analyzed have been methylated when a PCR product is obtained in the MSP method using such a primer set. The MSP method can also be carried out by using a primer set which does not allow for amplification of base sequences wherein cytosines at the CpG sites to be analyzed have not been converted into uracils, but which allows for amplification of base sequences wherein cytosines at the CpG sites have been converted into uracils. In this case, it can be understood that the CpG sites to be analyzed have been methylated when no PCR product is obtained.

In the methods provided herein, the presence or absence of PCR products can be confirmed, for example, by gel electrophoresis. The reaction solution after amplification may be subjected to electrophoresis in a gel to visually confirm whether or not a band derived from PCR products exists in the gel. Alternatively, an image of the gel after electrophoresis may be acquired for confirmation by image analysis. Image analysis can be conducted, for example, by acquiring a value which represents the contrast of pixels in a region where a PCR product is expected to exist (for example, band intensity) in an image of a gel and comparing this value with a predetermined threshold. Specifically, it can be determined that PCR products could be obtained when the value which represents the contrast of pixels is a predetermined threshold or higher, whereas it can be determined that no PCR product could be obtained when the value is lower than the predetermined threshold. The predetermined threshold for the contrast of pixels is not particularly limited. For example, the threshold may be either a value which represents the contrast of background pixels or a value twice or three times the value.

The respective primers contained in the primer set used in the MSP method can appropriately be designed by those skilled in the art according to the base sequence including the CpG sties to be analyzed. However, the primers are preferably designed so as to include cytosines at the CpG sites to be analyzed at the 3' end of the primers or in the vicinity thereof.

The methylation of CpG sites can also be analyzed using a microarray. In this case, the microarray for analysis can be prepared by fixing, on a substrate, a nucleic acid probe which is complementary to the base sequence of the promoter region of ZNF568 gene. Such a microarray can be prepared by a method which is known in the art.

In the microarray analysis, the DNA extracted from biological specimens is preferably labeled with a known labeling substance in the art. Accordingly, the determination method in this embodiment preferably further includes the step of labeling the extracted DNA. This labeling step is advantageously carried out after the above-described DNA amplification step since all the DNAs in the biological specimens can be labeled. Examples of the labeling substance include fluorescent materials, haptens such as biotin, and radioactive materials.

Examples of the fluorescent materials include Cy3, Cy5, FITC and Alexa Fluor™. Such DNA labeling facilitates measurement of signals from the probe on the microarray. The method for labeling DNA with these labeling substances is known in the art.

The above-described signals can be signals that are suitable depending on the type of microarray. For example, the signals may be electric signals which are generated in the presence of DNA fragments hybridized to the respective probes of the microarray, or may be fluorescence, luminescence, and other signals which are generated from labeling substances when the DNA to be analyzed is labeled in the above manner. Signals can be detected by means of a scanner which is equipped in a normal microarray measurement device. Examples of the scanner include GeneChip (registered trademark) Scanner 3000 7G (Affymetrix), and Illumina (registered trademark) BeadArray Reader (Illumina).

In the methods provided herein, when the analysis results of the presence of methylated CpG sites have been obtained by the above-described methylation analysis, it can be determined that there is methylation at the CpG sites that are present in the promoter region of the above-described gene. When the method involves determining the frequency of methylation and the result that the frequency of methylation obtained by the methylation analysis is equal to or higher than the predetermined threshold has been obtained, it can be determined that there is methylation at the CpG sites that are present in the promoter region of the above-described gene. The predetermined threshold is not particularly limited. The threshold can empirically be set based on the accumulated data on various biological specimens. Alternatively, the predetermined threshold may be set as follows. First, the frequency of methylation is analyzed for the DNAs extracted from biological specimens (normal stomach tissues or cells) which have preliminarily been confirmed to contain no cancer cell derived from gastric cancer and biological specimens containing gastric cancer-derived cancer cells. Then, a threshold is set from a range which is higher than the methylation frequency of the biological specimens containing no cancer cells and lower than that of the biological specimens containing cancer cells based on the analysis results obtained. A value which can highly accurately discriminate between the biological specimens containing no cancer cells and the biological specimens containing cancer cells is preferably defined as the threshold.

In particular embodiments of the methods provided herein, information on gastric cancer of a subject is acquired based on the determination results obtained in the above-described determination step. The information on gastric cancer is information as to whether or not the biological specimens collected from the subject contain gastric cancer-derived cancer cells. Alternatively, the information may be information which can serve as an index for diagnosis of gastric cancer or assists the diagnosis thereof. Preferably, the information is preferably information which indicates development or state, or both, of gastric cancer in a subject. Examples of such information include the possibility that gastric cancer has developed in a subject and the risk that gastric cancer will develop in a subject in the future, i.e. a prognosis on the development of cancer in the subject. When gastric cancer has developed in the subject, examples of the information on gastric cancer include the prognosis and cancer grade (stage) of the subject.

In the methods provided herein, when the presence of the methylated CpG sites has been determined in the determination step, information that the biological specimens contain cancer cells derived from gastric cancer is acquired. Alternatively, it is possible to acquire information which suggests the development of gastric cancer or that the gastric cancer state is not good (or bad). Also, it is possible to acquire information that the subject is highly likely to develop gastric cancer or that gastric cancer has developed already. If the subject has developed gastric cancer, it is possible to acquire information that the prognosis of the subject is not good (or bad) or that the cancer state is at a more advanced stage.

Conversely, when the absence of methylated CpG sites has been determined in the determination step, information that the biological specimens contain no cancer cells derived from gastric cancer is acquired. Accordingly, it is possible to acquire information which suggests there is no development of gastric cancer or that the gastric cancer state is good. Also, it is possible to acquire information that the subject is unlikely to develop gastric cancer or that gastric cancer has not developed yet. If the subject has developed gastric cancer, it is possible to acquire information that the prognosis of the subject is good or that the cancer state is at a not-so-much advanced stage.

The present disclosure also encompasses a nucleic acid to be used as a marker for acquiring information on gastric cancer through methylation analysis (hereinafter also referred to merely as "marker"). Such a nucleic acid can be an isolated nucleic acid containing at least one CpG site selected from CpG sites that are present in the promoter region of ZNF568 gene. As envisaged herein, methylation analysis can be conducted for the above-described marker in the DNA specimens prepared from the biological specimens collected from a subject, and information on gastric cancer of the subject can be acquired based on the analysis results obtained. The methylation analysis and acquisition of information on gastric cancer are as described previously.

The present disclosure also encompasses a nucleic acid having a sequence obtained by bisulfite treatment of a continuous base sequence of the entire or partial promoter region of ZNF568 gene, as the nucleic acid to be used as the above-described marker. In such a nucleic acid, the promoter region can include a CpG site and a cytosine base that does not constitute the CpG site. The cytosine base that does not constitute the CpG site may be any cytosine other than the cytosines contained in the CpG site, and examples of the cytosine include a cytosine in a base sequence (namely, CA, CT or CC) wherein cytosine (C) and adenine (A), thymine (T) or cytosine (C) are adjacent in this order in the 5' $\rightarrow$ 3' direction. Such a nucleic acid can be derived from a nucleic acid isolated from the subject.

In the methods envisaged herein, it is possible to perform methylation analysis for the above-described marker in the DNA specimens prepared from the biological samples collected from a subject and to acquire information on gastric cancer of the subject based on the analysis results obtained. The methylation analysis and acquisition of information on gastric cancer are also as described previously.

In the nucleic acid used as the marker as envisaged herein, non-methylated cytosines in the isolated DNA can be converted into uracils by the bisulfite treatment of the above-described isolated DNA, but methylated cytosines can be left as they are. Information on gastric cancer can then be acquired by methylation analysis of CpG sites in such a nucleic acid used as a marker. The above-described isolated DNA can be obtained as with the preparation of the DNA specimens described previously. The bisulfite treatment, methylation analysis and acquisition of information on gastric cancer are also as described previously.

The size of the nucleic acid used as a marker is not particularly limited so long as the size enables methylation analysis by the MSP method, sequence method or mass spectrometry. However, the size can be 50 bases to 200 bases, more preferably 80 bases to 130 bases.

Examples of the nucleic acid that can be used as a marker include those used as markers, which consist of the base sequence of SEQ ID NO: 2 or 3. The nucleic acids used as markers, which consist of the base sequence of SEQ ID NO: 2 or 3, are suitable for methylation analysis by the MSP method.

The present disclosure also encompasses a reagent kit for detection of gastric cancer (hereinafter also referred to merely as the "kit"). The kit as envisaged herein can include a primer for amplifying the bisulfite-treated nucleic acid. Such a primer can hybridize specifically to a nucleic acid consisting of a base sequence wherein cytosine that is present in a site other than a CpG site has been converted into another base in a base sequence having the CpG site in the promoter region of ZNF568 gene, and hybridizes specifically to a region including a methylated CpG site. The primer or primer set may hybridize to a promoter region (i.e. base sequence) including both a methylated CpG site and a converted cytosine (i.e. a cytosine that is present in a site other than a CpG site). The kit may also comprise a first primer which hybridizes specifically to a nucleic acid sequence including a methylated CpG site in the promoter region of ZNF568 gene.

In the kits envisaged herein, the primer included in the kit can be a primer for methylation analysis of CpG sites by the MSP method, an analysis method involving PCR amplification such as bisulfite sequence method, or mass spectrometry such as MassARRAY (registered trademark). The primer can preferably be a primer to be used in the MSP method or mass spectrometry such as MassARRAY (registered trademark). The base sequence of the primer can appropriately be set by those skilled in the art according to the base sequence of the promoter region described above.

Examples of such a primer include a primer of the base sequence of SEQ ID NO: 4 and a primer of the base sequence of SEQ ID NO: 5.

The above-described kit may include a second primer. Such a second primer can specifically hybridize to a nucleic acid which is a base sequence wherein cytosine has been converted into another base in a base sequence having a CpG site in the promoter region of ZNF568 gene. The second primer can specifically hybridize to a region including a sequence wherein cytosine at a non-methylated CpG site has been converted into another base by conversion treatment.

The present disclosure also encompasses a program product for allowing a computer to acquire information on gastric cancer of a patient. Examples of such a program product include programs which can be downloaded, for example, through the Internet and computer-readable recording media having the programs recorded therein.

There is exemplified a program for allowing a computer to execute the following steps of:

acquiring, from a measurement device, the analysis results of methylation of CpG sites that are present in the promoter region of ZNF568 gene contained in DNA specimens derived from a subject; and

determining the presence or absence of gastric cancer cells in the biological specimens based on the analysis results acquired.

One form of a device which is suitable for carry out the present method will now be described with reference to the drawings. However, the present disclosure is not limited hereto. Fig. 4 is a schematic view showing one example of a diagnosis auxiliary device for use in acquisition of information on gastric cancer of a subject. A diagnosis auxiliary device 10 shown in Fig. 4 includes a measurement device 20, and a determination device 30 that is connected to the measurement device 20.

When methylation analysis is performed by the MSP method, the measurement device 20 can be a gel imaging device such as a fluorescent image scanner. In this case, when a reaction solution which has been subjected to nucleic acid amplification by the MSP method is subjected to gel electrophoresis, and the gel after electrophoresis is set in the measurement device 20, the measurement device 20 detects an amplification product. The measurement device 20 acquires gel image information of the amplification product. The measurement device 20 provides the information acquired to the determination device 30.

The measurement device 20 may be a MALDI-TOF type mass spectrometer. In this case, the measurement device 20 acquires mass spectroscopic information including the time of flight and mass-to-charge ratio (m/z value) of a substance to be tested. When the specimens for measurement prepared from DNA specimens derived from a subject are set in the measurement device 20, the measurement device 20 acquires mass spectroscopic information of the nucleic acid contained in the specimens for measurement. The measurement device 20 then provides the mass spectroscopic information obtained to the determination device 30.

The determination device 30 includes a computer main body 300, an input unit 301 composed of a keyboard and/or a mouse, and a display unit 302 composed of LCD or CRT and displaying sample information, determination results and the like. The determination device 30 acquires from the measurement device 20 gel image information of the amplification product. Then, the determination device 30 executes a program which provides information on gastric cancer in a subject based on these pieces of information.

The determination device 30 may either be separated from the measurement device 20 as shown in Fig. 4, or include the measurement device 20. In the latter case, the determination device 30, itself, can be the diagnosis auxiliary device 10.

Fig. 5 is a block diagram showing the software of the computer main body 300 of the determination device 30 as function blocks. The computer is provided with an acquisition unit 321, a storage unit 322, a calculation unit 323, a determination unit 324, and an output unit 325, as shown in Fig. 5. The acquisition unit 321 is connected, through a network, to the measurement device 20 so as to be able to communicate with the device.

The acquisition unit 321 acquires the information provided from the measurement device 20. The storage unit 322 stores formulae and/or processing programs for acquiring the threshold and band intensity required for determination therein. The calculation unit 323 calculates the band intensity in accordance with the stored processing program using the information acquired by the acquisition unit 321. The determination unit 324 determines whether or not the band intensity acquired by the acquisition unit 321 or calculated by the calculation unit 323 is equal to or higher than the threshold stored in the storage section 322. The output unit 325 outputs the determination results by the determination unit 324 to the display unit 302 as information on gastric cancer of a subject (for example, the presence or absence of gastric cancer cells in the biological specimens collected from the subject).

Fig. 6 is a block diagram showing the hardware configuration of the computer main body 300 shown in Fig. 5. The computer main body 300 is provided with a CPU (Central Processing Unit) 310, a ROM (Read Only Memory) 311, a RAM (Random Access Memory) 312, a hard disk 313, and an input/output interface 314, a readout device 315, a communication interface 316, and an image output interface 317, as shown in Fig. 6. The CPU 310, ROM 311, RAM 312, hard disk 313, input/output interface 314, readout device 315, communication interface 316, and image output interface 317 are connected by a bus 318 so as to be capable of data communication.

The CPU 310 can execute programs stored in the ROM 311 and programs loaded in the RAM 312. The execution of programs by the CPU 310 results in the execution of the respective functions shown in Fig. 5. Thus, the determination device 30 functions as a determination device for providing information on gastric cancer of a subject.

The ROM 311 is composed of mask ROM, PROM, EPROM, EEPROM and the like. The ROM 311 has recorded therein programs to be executed by the CPU 310 as described above and data to be used for execution of these programs.

The RAM 312 is composed of SRAM, DRAM and the like. The RAM 312 is used in readout of programs recorded in the ROM 311 and the hard disk 313. The RAM 312 is also utilized as a working area of the CPU 310 when these programs are executed.

The hard disk 313 has installed therein an operating system to be executed by the CPU310, programs such as an application program (computer program for providing information on gastric cancer of a subject), and data to be used for execution of the programs.

The readout device 315 is composed of a flexible disk drive, a CD-ROM drive, a DVD-ROM drive and the like. The readout device 315 can read out programs or data stored in a portable recording medium 40.

The input/output interface 314 is composed, for example, of serial interfaces such as USB, IEEE1394 and RS-232C, parallel interfaces such as SCSI, IDE and IEEE1284, and analog interfaces including, for example, a D/A converter and an A/D converter. To the input/output interface 314, the input unit 301 such as a keyboard and/or a mouse is connected. Operators can input various commands to the computer main body 300 by means of the input unit 301.

The communication interface 316 is, for example, an Ethernet (registered trademark) interface. The computer main body 300 can also transmit printing data, for example, to a printer by means of the communication interface 316.

The image output interface 317 is connected to the display unit 302 which is composed of LCD, CRT or the like. Thus, the display unit 302 can output a video signal in accordance with the image data given from the CPU 310. The display unit 302 displays an image (screen) in accordance with the input video signal.

Next, the processing procedures for acquiring information on gastric cancer of a subject by the diagnosis auxiliary device 10 will be described.

Fig. 7 shows one example of a flow chart for acquiring information on gastric cancer. Explanation is made herein about an example case where band intensity is acquired from the gel image information obtained using biological specimens derived from a subject to determine whether or not the obtained band intensity is equal to or higher than the threshold. The present disclosure, however, is not limited to this example alone.

First, in Step S1-1, the acquisition unit 321 of the diagnosis auxiliary device 10 acquires gel image information on ZNF568 gene from the measurement device 20. Next, in Step S1-2, the calculation unit 323 acquires band intensity from the obtained gel image information, and the calculation unit 323 transmits the band intensity to the storage unit 322.

Thereafter, in Step 1-3, the determination unit 324 determines whether or not the band intensity acquired in Step S1-2 is equal to or higher than the threshold stored in the storage unit 322. When the band intensity is equal to or higher than the threshold, the routine proceeds to Step S1-4. Then, the determination unit 324 transmits to the output unit 325 the determination results which indicate that gastric cancer cells exist in the biological specimens derived from the subject. On the other hand, when the band intensity is lower than the threshold, the routine proceeds to Step S1-5. Then, the determination unit 324 transmits to the output unit 325 the determination results which indicate that no gastric cancer cell exists in the biological specimens derived from the subject.

Finally, in Step S1-6, the output unit 325 outputs the determination results as information on gastric cancer of the subject to allow the display unit 302 to display the information. Thus, the diagnosis auxiliary device 10 can provide the information which assists the diagnosis as to whether or not the subject suffers from gastric cancer to physicians and the like.

Hereinafter, the present invention will be described in detail by way of examples. However, the present invention is not limited to these examples.

## EXAMPLES

### Example 1: Identification of novel marker utilizing methylation data for cancer part tissues and non-cancer part tissues of gastric cancer and normal tissues

#### (1) Acquisition of methylation data

In Example 1, methylation data of Infinium HumanMethylation450 BeadChip (Illumina) published on TCGA (The Cancer Genome Atlas: http://tcga-data.nci.nih.gov/tcga/tcgaHome2.jsp) was acquired for cancer part tissues (69 samples) and non-cancer part tissues (2 samples) of gastric cancer. For normal tissues (23 samples), methylation data of Infinium HumanMethylation450 BeadChip published in the document of Nazor KL. et al. (Recurrent variations in DNA methylation in human pluripotent stem cells and their differentiated derivatives. Cell Stem Cell 2012;10(5):620-634) was acquired.

#### (2) Identification of novel marker

As a result of data mining by use of Infinium HumanMethylation450 BeadChip (Illumina), the promoter regions of ZNF568 genes were identified as markers in which methylation was observed specifically for cancer part tissues of gastric cancer (see Fig. 1). Hereinafter, these markers are referred to also as markers of the present example.

### Example 2: Comparison of methylation data among plurality of types of cancer/tumor-derived cancer/tumor tissue samples, non-cancer part tissue samples, and normal tissue samples

#### (1) Acquisition of methylation data

Methylation data for 14 types of cancer/tumor tissue samples, 11 types of non-cancer part tissue samples, and 19 types of normal tissue samples was compared in Example 2. The numbers of the respective tissue samples are indicated in the following table.

[Table 2]

| Cancer/tumor sample | |
|---|---|
| Tissue | Number of samples |
| Cerebral tumor | 114 |
| Lung cancer | 370 |
| Breast cancer | 548 |
| Gastric cancer | 69 |
| Liver cancer | 81 |
| Kidney cancer | 363 |
| Bladder cancer | 78 |
| Cancer of uterine body | 334 |
| Ovarian cancer | 49 |
| Prostate cancer | 153 |
| Acute leukemia | 192 |
| Sarcoma | 73 |
| Malignant melanoma | 242 |
| Thyroid cancer | 316 |

[Table 3]

| Non-cancer tissue | |
|---|---|
| Tissue | Number of samples |
| Cerebral tumor | 1 |
| Lung cancer | 75 |
| Breast cancer | 98 |
| Gastric cancer | 2 |
| Hepatoma | 14 |
| Kidney cancer | 208 |
| Bladder cancer | 15 |
| Cancer of uterine body | 36 |
| Ovarian cancer | 1 |
| Prostate cancer | 49 |
| Thyroid cancer | 36 |

[Table 4]

| Normal tissue | | | | |
|---|---|---|---|---|
| Tissue | RCAST | Article 1 | Article 2 | Total |
| Normal Brain (Brain) | 2 | 1 | 0 | 3 |

(continued)

| Normal tissue | | | | |
|---|---|---|---|---|
| Tissue | RCAST | Article 1 | Article 2 | Total |
| Normal Oral (Oral) | 2 | 0 | 0 | 2 |
| Normal Lung (Lung) | 0 | 2 | 0 | 2 |
| Normal Colon Mucosa (Colon) | 2 | 0 | 0 | 2 |
| Normal Liver (Liver) | 2 | 0 | 0 | 2 |
| Peripheral Blood of Healthy Person (Blood) | 2 | 2 | 0 | 4 |
| Normal Skeletal Muscle (Skeletal) | 2 | 2 | 0 | 4 |
| Normal Testis (Testis) | 1 | 0 | 0 | 1 |
| Normal Stomach Mucosa (Stomach) | 0 | 1 | 0 | 1 |
| Normal Pancreas (Pancreas) | 0 | 2 | 0 | 2 |
| Normal Spleen (Spleen) | 0 | 2 | 0 | 2 |
| Normal Kidney (Kidney) | 0 | 0 | 0 | 0 |
| Normal Adrenal Gland (Adrenal gland) | 0 | 2 | 0 | 2 |
| Normal Ureter (Ureter) | 0 | 2 | 0 | 2 |
| Normal Bladder (Bladder) | 0 | 2 | 0 | 2 |
| Normal Lymph Nodes (Lymph nodes) | 0 | 2 | 0 | 2 |
| Normal Adipose Tissue (Adipose tissue) | 0 | 2 | 0 | 2 |
| Normal Heart (Heart) | 0 | 1 | 0 | 1 |
| Various Normal Blood Cell Ingredients (WB, PBMC, Gran, CD4+, CD8+, CD14+, CD19+, CD56+, Neu, Eos) | 0 | 0 | 60 | 60 |

For the samples in the column "RCAST" in Table 4, the present inventors acquired the methylation data by the Infinium Methylation Assay using Infinium HumanMethylation450 BeadChip (Illumina). For the samples in the columns "Article 1" and "Article 2," the methylation data of Infinium HumanMethylation450 BeadChip (Illumina) published in the following documents was acquired.

- Article 1: Nazor KL et al., Recurrent variations in DNA methylation in human pluripotent stem cells and their differentiated derivatives. Cell Stem Cell 2012; 10(5): 620-634
- Article 2: Reinius LE et al., Differential DNA Methylation in Purified Human Blood Cells: Implications for Cell Lineage and Studies on Disease Susceptibility, PLoS One, 7 (7) e41361

The methylation data as used herein is a methylation rate (mCpG) of the respective CpG regions of ZNF568 obtained as follows. Infinium HumanMethylation450 BeadChip is provided with probes for methylation and probes for non-methylation for each of 482,421 CpG sites among the CpG sites in the human genome. The signal intensity (signal M) of the probes for methylation for the CpG sites of the target genes and the signal intensity (signal U) of the probes for non-methylation were detected by BeadArray Reader to calculate the methylation rates (mCpG) of the CpG regions of the target genes by the following calculation formula.

$$(\text{mCpG}) = (\text{signal M}) / \{(\text{signal M}) + (\text{signal U})\}$$

(2) Comparison of methylation positive rates among caner/tumor tissue sample, non-cancer part tissue sample, and normal tissue sample

When a statistically significant difference between the tumor tissue samples and the normal tissue samples was observed for the methylation rate (mCpG) obtained, the samples were defined as being methylation positive. The methylation positive rates (%) for the various types of cancer were calculated.

$$\text{Methylation positive rate (\%)} = (\text{number of methylation-positive samples / total number of samples}) \times 100$$

(For example, in the case of cerebral tumors, the methylation positive rate is calculated by the formula: "number of methylation-positive samples among cerebral tumor samples / total number of cerebral tumor samples (= 114) × 100.")

The results are shown in Fig. 2. In this figure, the "normal tissue" represents normal tissues except 60 samples of various normal blood cell ingredients among the tissues indicated in Table 4, and the "normal blood cell" represents 60 samples of various normal blood cell ingredients. From Fig. 2, all the markers according to the present example hardly exhibit methylation in the non-cancer part tissues as well as the human normal tissues and human normal blood cells. All the markers according to the present example were found to have been particularly highly methylated in gastric cancer as compared with other types of cancer. Accordingly, it was shown that the markers of the present example are suitable for detection of gastric cancer.

Example 3: Comparison of methylation data (MSP) between tissues of healthy person and patient with gastric cancer

(1) Biological sample

In Example 3, FFPE samples (6 samples) of cancer part tissues collected from patients with gastric cancer were used as biological specimens derived from the patients. As control specimens, normal stomach tissues (2 samples) were used.

(2) Preparation of specimen for measurement

(i) Extraction of genome DNA

The genome DNA was extracted from the respective FFPE samples of the respective gastric cancer tissues described above using QIAamp DNA FFPE Tissue Kit (QIAGEN). The genome DNA was extracted from the normal tissues using QIAamp DNA Mini Kit (QIAGEN). As control genome DNA, the genome DNA of human peripheral blood lymphocytes was used. This genome DNA of human peripheral blood lymphocytes was amplified using GenomiPhi v2DNA amplification Kit (GE Healthcare Life Sciences). The resultant amplification product is composed of non-methylated DNA. Next, the amplification product was fragmented by Bioruptor (manufactured by COSMO BIO) to obtain a solution of non-methylated DNA fragments (0% methylated DNA). A part of the solution of this non-methylated DNA fragments was taken. SssI methylase (New England Biolab) was reacted with this for methylation of all the cytosines contained in the CG sequence, thereby obtaining a solution of methylated DNA fragments (100% methylated DNA).

(ii) Bisulfite Treatment

The respective DNA fragments obtained above (500 ng) were subjected to bisulfite treatment using EZ DNA Methylation Kit (Zymo Research), and the treated genome DNA was eluted in sterile distilled water (80 μl).

(3) MSP

The specimens for measurement and control specimens obtained in the above (2) were used for MSP. The compositions of the PCR reagents used, primer sets and PCR conditions are indicated as follows.

<PCR reagent>

| | |
|---|---|
| DW (sterile water) | 18.8 μL |
| 10×PCR buffer with $MgCl_2$ (Roche) | 2.5 μL |
| 10 mM dNTP mix | 0.5 μL |
| 10 μM sense primer | 1.0 μL |

(continued)

| | |
|---|---|
| 10 μM antisense primer | 1.0 μL |
| Faststart Taq polymerase(Roche) | 0.2 μL |
| Specimen for measurement | 1.0 μL |
| Total | 25.0 μL |

<Primer set>

The primer sets used in the above-described MSP are indicated in Table 5. This primer set is a primer set that can provide an amplification product when DNA in a region to be amplified has been methylated (hereinafter, also referred to as "primer set for the detection of methylation"). A primer set for determining whether or not the bisulfite treatment had been properly conducted was also used as a primer set for the management of accuracy (see Table 6). The base sequence of the region to be analyzed by the primer set for the detection of methylation in the promoter region of ZNF568 gene is indicated by SEQ ID NO: 2.

[Table 5]

| Gene Name | Base Sequence | SEQ ID | PCR Product (bp) | Annealing Temperature (X) | Cycle (Y) | Region to Be Amplified |
|---|---|---|---|---|---|---|
| ZNF568 | TTTGTTATAGATTTATTTGCGGGTC | 4 | 120 | 60 | 38 | chr19:37, 407, 286-37, 407,405 |
| | TACTCGCCCCCTACTACGAA | 5 | | | | |

[Table 6]

| Primer for Management of Accuracy | Base Sequence | SEQ ID | PCR Product (bp) | Annealing Temp (X) | Cycle (Y) |
|---|---|---|---|---|---|
| Forward | GGGATATTAAGTGGAGTTATTTTGGTTTTAGTT | 6 | 129 | 60 | 40 |
| Reverse | CCCTCCCAACATCCTTCCTAA | 7 | | | |

<PCR reaction condition>

The specimens were subjected to MSP at 95°C for 6 minutes;
in Y cycle(s) of at 95°C for 30 seconds, at X°C for 30 seconds, and at 72°C for 30 seconds; and
at 72°C for 7 minutes; and then
allowed to stand at 16°C.

In the above-described reaction conditions, "X" and "Y" represent the annealing temperature and number of cycles, respectively, indicated in Tables 5 and 6.

(4) Analysis of results of methylation-specific PCR (MSP)

The amplification products obtained by the above-described MSP were confirmed through 2% agarose gel electrophoresis. The results are shown in Fig. 4. In this figure, the numerical values "0" and "100," which are shown as "control," represent 0% methylated control specimens and 100% methylated control specimens, respectively.

From Fig. 3, bands were detected for all the specimens in PCR using the primer sets for the management of accuracy. This reveals that the specimens were properly bisulfite-treated. No band derived from methylated CpG was detected for the specimens of normal stomach tissues in the PCR using the primer set for detection of methylation. On the other hand, bands were detected for ZNF568 in 6 of 6 cases in the PCR for the samples of gastric cancer tissues. The methylation analysis of the markers of the present example by the MSP method indicated the correlation between the methylation of the markers of the present example and gastric cancer, as with the results of the Infinium method in Example 1. In other words, ZNF568 is a highly specific marker that exhibits a methylation tendency, in a high rate, in gastric cancer, but that would not be detected in normal stomach tissues.

## Claims

**1.** A method for acquiring information on the presence of gastric cancer in a subject, the method comprising the steps of:

analyzing, in a DNA specimen prepared from a biological sample of said subject, the methylation status of a CpG site in a promoter region of the ZNF568 gene; and

acquiring information that the biological specimen comprises a gastric cancer cell when it is determined that the methylation is present.

2. The method according to claim 1, wherein the methylation status of SEQ ID NO: 2 is analyzed in the analyzing step.

3. The method according to claim 1 or 2, wherein the analyzing step is conducted by at least one method selected from the group consisting of mass spectrometry and methylation-specific PCR.

4. The method according to any one of claims 1 to 3, wherein the biological sample is a tissue, blood or serum.

5. The method according to any one of claims 1 to 4, wherein
the analyzing step comprises: calculating methylation frequency of the promoter region, and
the acquiring step comprises: comparing the frequency with a predetermined threshold; and acquiring information that the biological specimen comprises the gastric cancer cell when the frequency is equal to or higher than the threshold.

6. The method according to any one of claims 1 to 5, which further comprises the step of preparing the DNA specimen from a biological sample of said subject.

7. The method according to claim 6, wherein the preparing step comprises treating DNA in the biological specimen with bisulfite.

8. Use of a reagent kit for the detection of gastric cancer,
wherein the reagent kit comprises a primer capable of specifically hybridizing to a promoter region of ZNF568 gene, wherein the promoter region comprises: a uracil base converted from unmethylated cytosine base by a bisulfite treatment and a methylated cytosine base, and
wherein the primer is used for analyzing methylation status of a CpG site in the promoter region of ZNF568 gene contained in a DNA specimen.

9. Use according to claim 8, wherein the primer consists of the base sequence of SEQ ID NO:4 or 5.

## Patentansprüche

1. Verfahren zur Erfassung von Informationen über das Vorhandensein von Magenkrebs bei einem Individuum, wobei das Verfahren die Schritte umfasst:

   Analysieren des Methylierungsstatus einer CpG-Stelle in einer Promotorregion des ZNF568-Gens in einer aus einer biologischen Probe des Individuums präparierten DNA-Probe; und
   Erfassen von Informationen, dass die biologische Probe eine Magenkrebszelle umfasst, wenn bestimmt wird, dass die Methylierung vorliegt.

2. Verfahren nach Anspruch 1, wobei der Methylierungsstatus von SEQ ID NO: 2 im Analyseschritt analysiert wird.

3. Verfahren nach Anspruch 1 oder 2, wobei der Analyseschritt durch mindestens ein Verfahren durchgeführt wird, ausgewählt aus der Gruppe bestehend aus Massenspektrometrie und methylierungsspezifischer PCR.

4. Verfahren nach einem der Ansprüche 1 bis 3, wobei die biologische Probe ein Gewebe, Blut oder Serum ist.

5. Verfahren nach einem der Ansprüche 1 bis 4, wobei
der Analyseschritt umfasst: Berechnen der Methylierungsfrequenz der Promotorregion, und
der Erfassungsschritt umfasst: Vergleichen der Frequenz mit einem vorbestimmten Schwellenwert; und Erfassen von Informationen, dass die biologische Probe die Magenkrebszelle umfasst, wenn die Frequenz gleich oder höher als der Schwellenwert ist.

6. Verfahren nach einem der Ansprüche 1 bis 5, das zudem den Schritt der Herstellung der DNA-Probe aus einer biologischen Probe des Individuums umfasst.

7. Verfahren nach Anspruch 6, wobei der Präparationsschritt das Behandeln von DNA in der biologischen Probe mit Bisulfit umfasst.

8. Verwendung eines Reagenzienkits zum Nachweis von Magenkrebs,
wobei das Reagenzienkit einen Primer umfasst, der spezifisch an eine Promotorregion des ZNF568-Gens hybridisieren kann, wobei die Promotorregion umfasst: eine Uracilbase, die durch eine Bisulfitbehandlung aus einer unmethylierten Cytosinbase umgewandelt wurde, und eine methylierte Cytosinbase, und
wobei der Primer zur Analyse des Methylierungsstatus einer CpG-Stelle in der Promotorregion des in der DNA-Probe enthaltenen ZNF568-Gens verwendet wird.

9. Verwendung nach Anspruch 8, wobei der Primer aus der Basensequenz von SEQ ID NO: 4 oder 5 besteht.

**Revendications**

1. Procédé pour acquérir des informations sur la présence d'un cancer gastrique chez un sujet, le procédé comprenant les étapes :

   d'analyse, dans un spécimen d'ADN préparé à partir d'un échantillon biologique dudit sujet, du statut de méthylation d'un site CpG dans une région de promoteur du gène ZNF568 ; et
   d'acquisition d'informations selon lesquelles le spécimen biologique comprend une cellule de cancer gastrique lorsqu'il est déterminé que la méthylation est présente.

2. Procédé selon la revendication 1, dans lequel le statut de méthylation de SEQ ID n° 2 est analysé à l'étape d'analyse.

3. Procédé selon la revendication 1 ou 2, dans lequel l'étape d'analyse est conduite par au moins un procédé choisi dans le groupe consistant en la spectrométrie de masse et de la PCR spécifique à la méthylation.

4. Procédé selon l'une quelconque des revendications 1 à 3, dans lequel l'échantillon biologique est un tissu, du sang ou du sérum.

5. Procédé selon l'une quelconque des revendications 1 à 4, dans lequel
l'étape d'analyse comprend : le calcul de la fréquence de méthylation de la région de promoteur, et
l'étape d'acquisition comprend : la comparaison de la fréquence à un seuil prédéterminé ; et l'acquisition d'informations selon lesquelles le spécimen biologique comprend la cellule de cancer gastrique lorsque la fréquence est supérieure ou égale au seuil.

6. Procédé selon l'une quelconque des revendications 1 à 5, qui comprend en outre l'étape de préparation du spécimen d'ADN à partir d'un échantillon biologique dudit sujet.

7. Procédé selon la revendication 6, dans lequel l'étape de préparation comprend le traitement de l'ADN dans le spécimen biologique avec du bisulfite.

8. Utilisation d'un kit de réactif pour la détection d'un cancer gastrique,
dans laquelle le kit de réactif comprend une amorce capable de s'hybrider spécifiquement à une région de promoteur du gène ZNF568, dans laquelle la région de promoteur comprend :

   une base uracile convertie à partir d'une base cytosine non méthylée par un traitement au bisulfite et une base cytosine méthylée, et
   dans laquelle l'amorce est utilisée pour analyser le statut de méthylation d'un site CpG dans la région de promoteur du gène ZNF568 contenu dans un spécimen d'ADN.

9. Utilisation selon la revendication 8, dans laquelle l'amorce consiste en la séquence de base de SEQ ID n° 4 ou 5.

*FIG. 1*

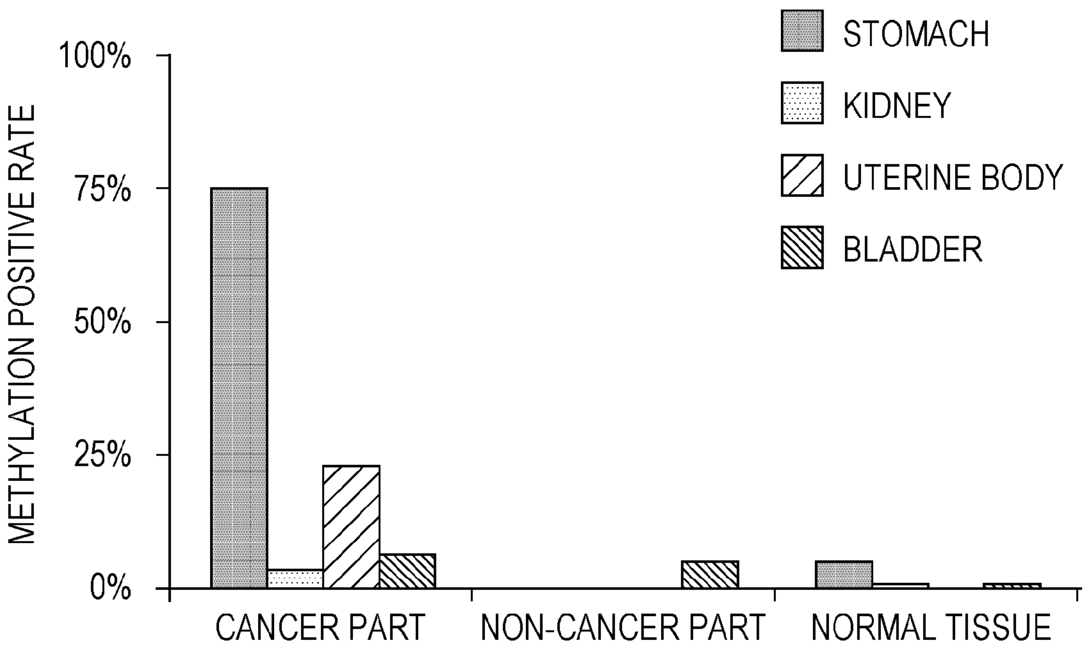
STOMACH
KIDNEY
UTERINE BODY
BLADDER
100%
75%
50%
25%
0%
METHYLATION POSITIVE RATE
CANCER PART
NON-CANCER PART
NORMAL TISSUE

FIG. 2

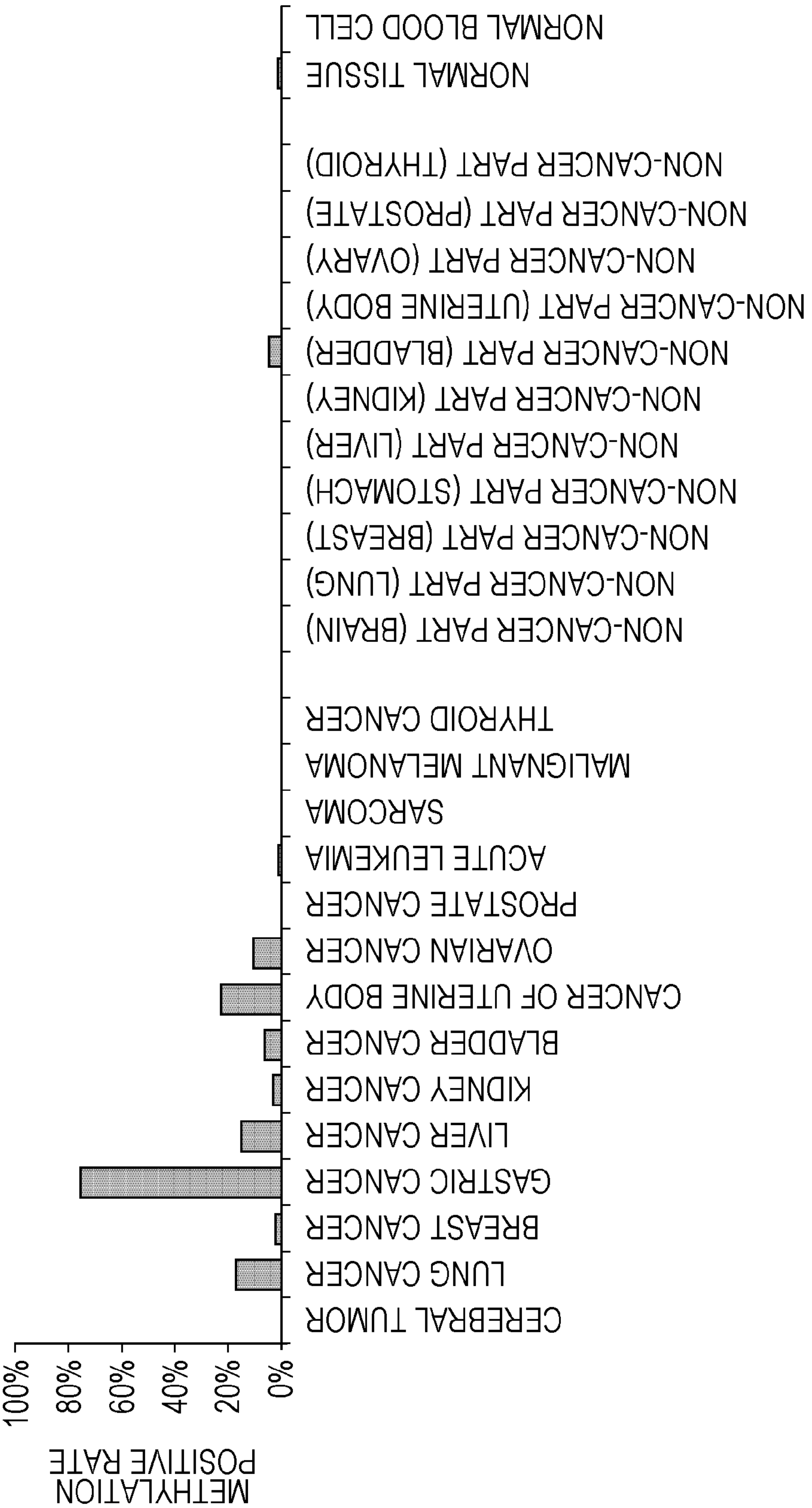
METHYLATION POSITIVE RATE
0%
20%
40%
60%
80%
100%
CEREBRAL TUMOR
LUNG CANCER
BREAST CANCER
GASTRIC CANCER
LIVER CANCER
KIDNEY CANCER
BLADDER CANCER
CANCER OF UTERINE BODY
OVARIAN CANCER
PROSTATE CANCER
ACUTE LEUKEMIA
SARCOMA
MALIGNANT MELANOMA
THYROID CANCER
NON-CANCER PART (BRAIN)
NON-CANCER PART (LUNG)
NON-CANCER PART (BREAST)
NON-CANCER PART (STOMACH)
NON-CANCER PART (LIVER)
NON-CANCER PART (KIDNEY)
NON-CANCER PART (BLADDER)
NON-CANCER PART (UTERINE BODY)
NON-CANCER PART (OVARY)
NON-CANCER PART (PROSTATE)
NON-CANCER PART (THYROID)
NORMAL TISSUE
NORMAL BLOOD CELL

FIG. 3

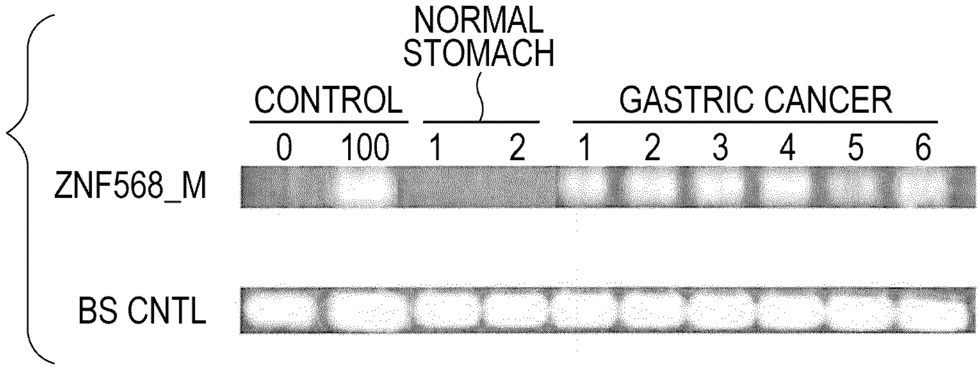
NORMAL STOMACH
CONTROL
GASTRIC CANCER
0
100
1
2
1
2
3
4
5
6
ZNF568_M
BS CNTL

FIG. 4

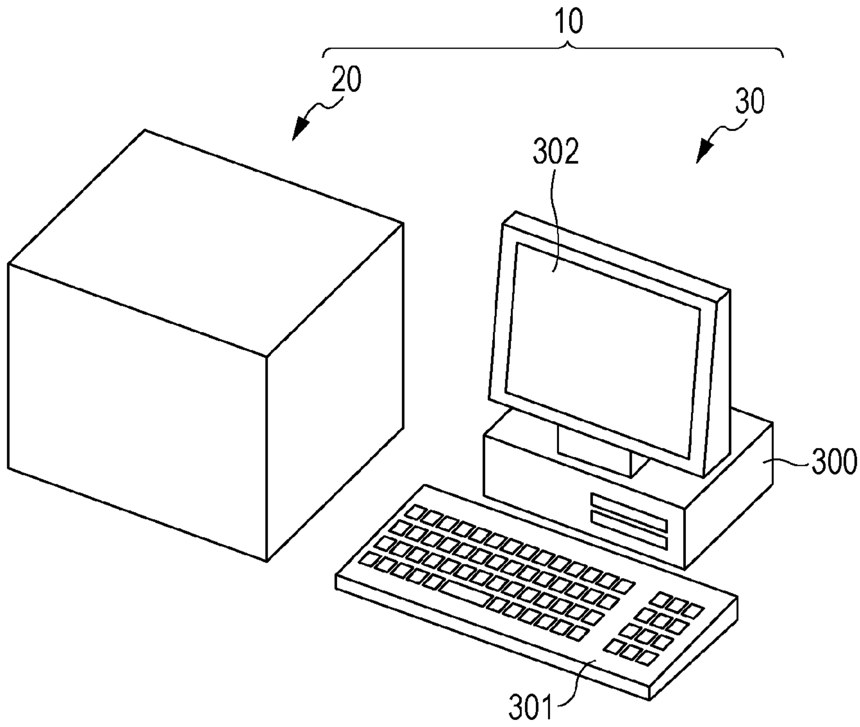
10
20
30
302
300
301

FIG. 5

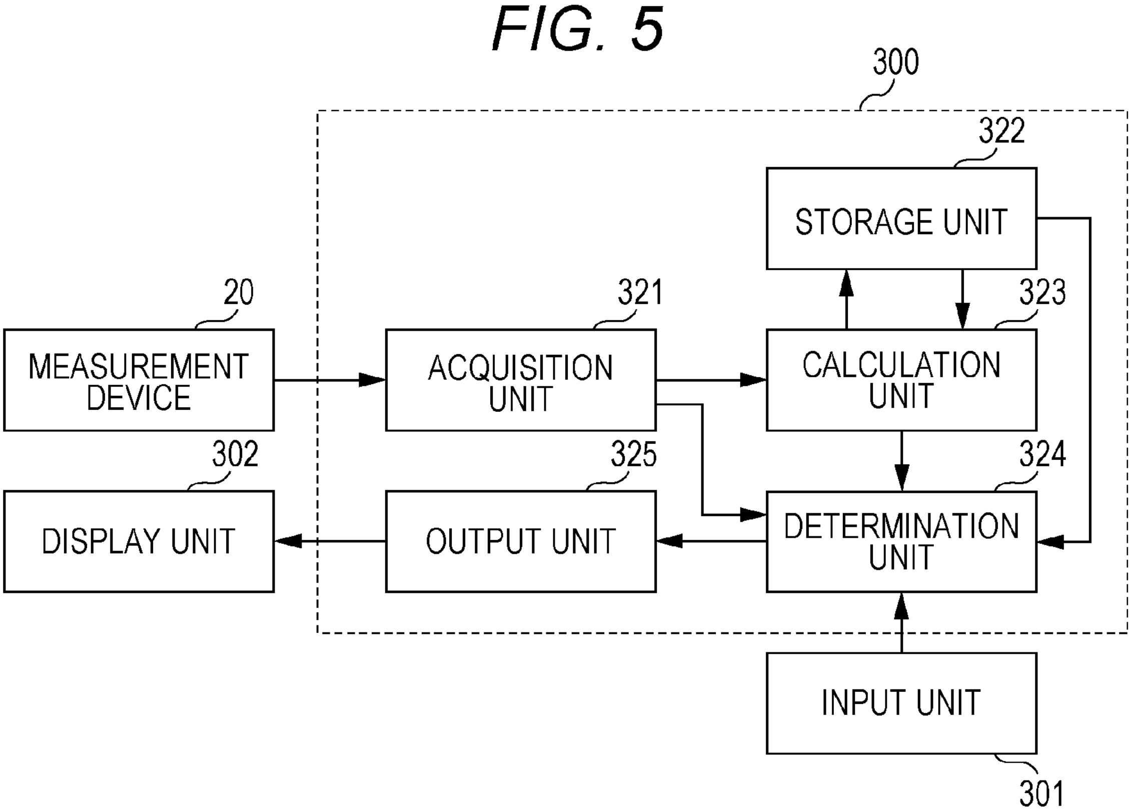
300
322
STORAGE UNIT
20
321
323
MEASUREMENT DEVICE
ACQUISITION UNIT
CALCULATION UNIT
302
325
324
DISPLAY UNIT
OUTPUT UNIT
DETERMINATION UNIT
INPUT UNIT
301

FIG. 6

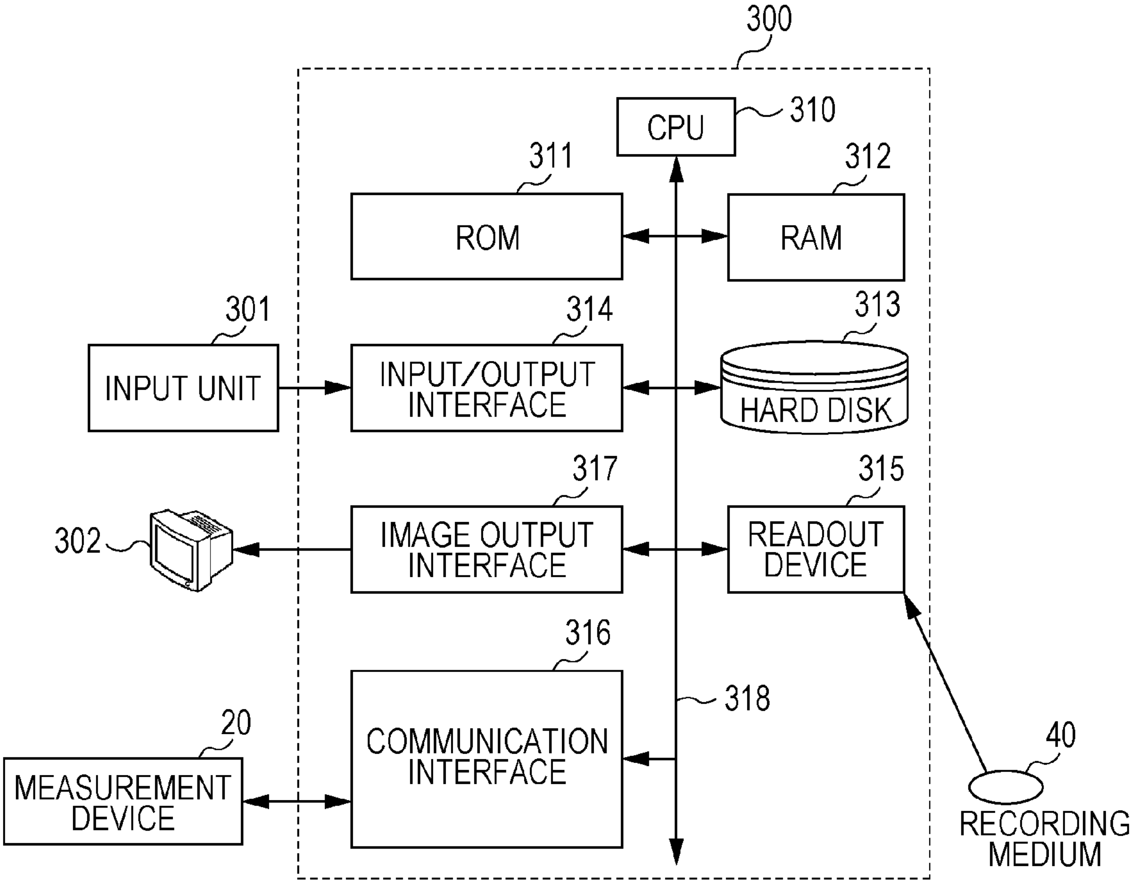
300
CPU
310
311
312
ROM
RAM
301
314
313
INPUT UNIT
INPUT/OUTPUT INTERFACE
HARD DISK
317
315
302
IMAGE OUTPUT INTERFACE
READOUT DEVICE
316
318
20
40
COMMUNICATION INTERFACE
MEASUREMENT DEVICE
RECORDING MEDIUM

FIG. 7

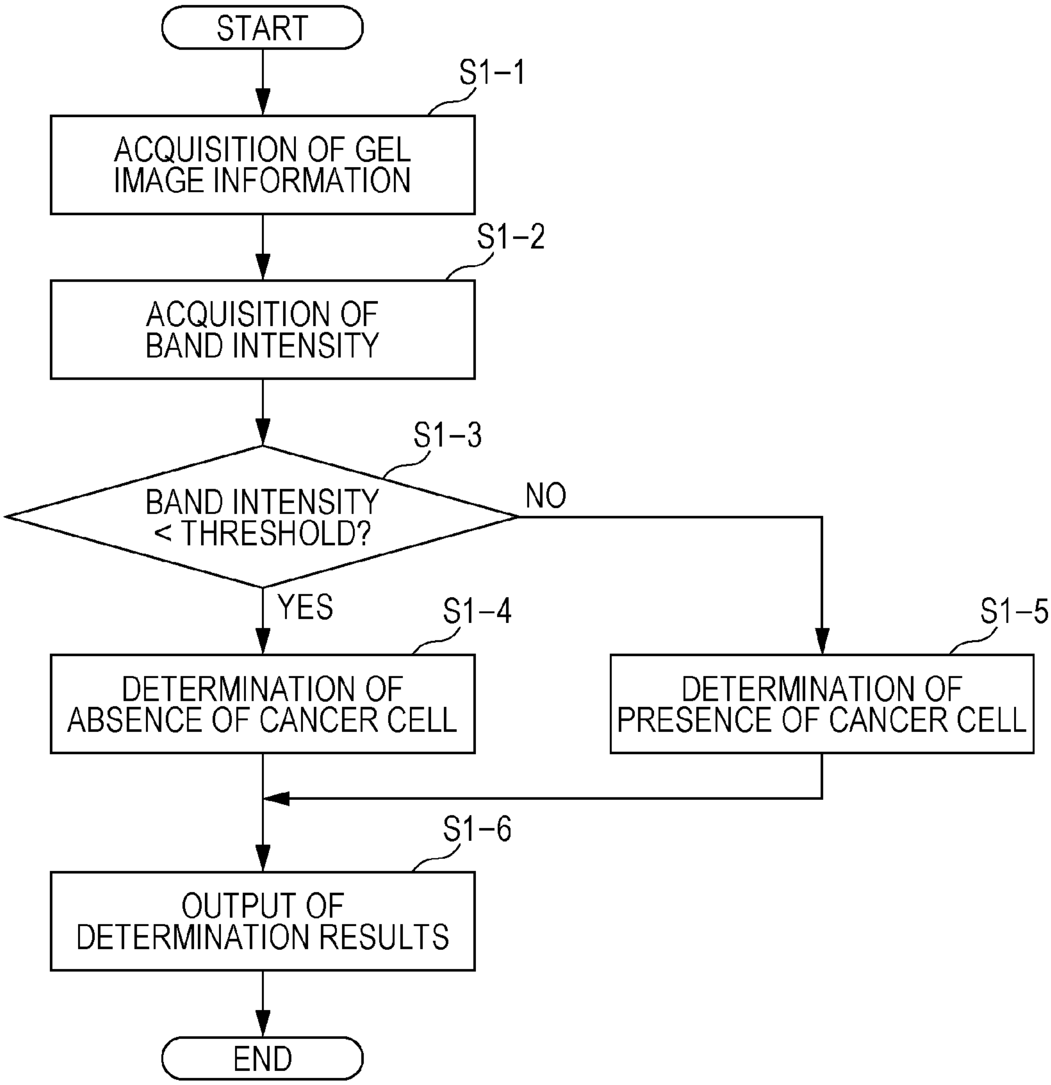
START
S1-1
ACQUISITION OF GEL IMAGE INFORMATION
S1-2
ACQUISITION OF BAND INTENSITY
S1-3
BAND INTENSITY < THRESHOLD?
NO
YES
S1-4
DETERMINATION OF ABSENCE OF CANCER CELL
S1-5
DETERMINATION OF PRESENCE OF CANCER CELL
S1-6
OUTPUT OF DETERMINATION RESULTS
END

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013097868 A
- WO 2014062218 A
- WO 2012070861 A
- JP 2015073624 A


### Non-patent literature cited in the description


- **HRASOVEC S. et al.** *Disease Markers,* 2013, vol. 34, 93-104
- **NAZOR KL. et al.** Recurrent variations in DNA methylation in human pluripotent stem cells and their differentiated derivatives. *Cell Stem Cell,* 2012, vol. 10 (5), 620-634
- **NAZOR KL et al.** Recurrent variations in DNA methylation in human pluripotent stem cells and their differentiated derivatives. *Cell Stem Cell,* 2012, vol. 10 (5), 620-634
- **REINIUS LE et al.** Differential DNA Methylation in Purified Human Blood Cells: Implications for Cell Lineage and Studies on Disease Susceptibility. *PLoS One,* vol. 7 (7), e41361